# EUROPEAN PATENT APPLICATION

(11) **EP 3 245 954 A1**
(43) Date of publication of application: **22.11.2017**
(21) Application number: 15877942.1
(22) Date of filing: 10.11.2015
(51) Int. Cl.: A61B 8/12

(54) **ULTRASONIC OBSERVATION SYSTEM**

(30) Priority: 16.01.2015 JP 2015006752
(71) Applicant: Olympus Corporation, Hachioji-shi Tokyo 192-8507 (JP)
(72) Inventor: YOSHIMURA, Takehiro, Tokyo 192-8507, (JP)
(74) Representative: Gunzelmann, Rainer
(86) International application number: PCT/JP2015/081605
(87) International publication number: WO 2016/113990

(57) **Abstract**

An ultrasound observation system according to the present invention includes an image processing unit, a display unit, a multiple input reception unit, an input information determination unit, an operation mode setting unit, and a target region setting unit. The image processing unit generates an ultrasound image based on an ultrasound signal obtained by an ultrasound endoscope configured to transmit ultrasound to a subject as an observation target and receive the ultrasound reflected from the subject. The display unit displays the ultrasound image generated by the image processing unit. The multiple input reception unit is configured to simultaneously receive inputs at multiple points on the ultrasound image displayed on the display unit. The input information determination unit determines the number of inputs received by the multiple input reception unit and positions of the inputs. The operation mode setting unit sets an operation mode related to the ultrasound image based on a result of determination by the input information determination unit. The target region setting unit sets a target region to be processed corresponding to the operation mode set by the operation mode setting unit, based on the positions of the inputs determined by the input information determination unit.

## Description

### Field

The present invention relates to an ultrasound observation system configured to observe an observation target using ultrasound.

### Background

Ultrasound is applied in some cases for observing characteristics of a living tissue or material as an observation target. Specifically, ultrasound transmitted toward the observation target is reflected as an ultrasound echo from the observation target, and predetermined signal processing is performed on the reflected ultrasound echo, whereby information related to characteristics of the observation target is obtained.

Ultrasound diagnosis of a living tissue, or the like, inside the body uses an ultrasound endoscope in which an ultrasound transducer is provided at a distal end of an insertion unit. A practitioner including a doctor inserts an insertion unit into the body and operates an operating unit at hand, whereby the ultrasound transducer obtains an ultrasound echo and diagnosis is performed on the basis of information based on the ultrasound echo (ultrasound image).

At the time of diagnosis, a plurality of obtained ultrasound images is displayed on a monitor in time series. At this time, a practitioner performs diagnosis of the ultrasound image by performing input of an instruction of setting processing and measurement processing of the examination region. As a diagnosis system for performing such diagnosis, there is a disclosed technique capable of performing direct input of an instruction on the ultrasound image using a touch panel (for example, refer to Patent Literature 1). According to Patent Literature 1, setting of operation modes such as a region setting mode is performed in accordance with the operation position on the touch panel, and region setting processing corresponding to the operation position is performed by touch panel operation after a change to the region setting mode.

### Citation List

### Patent Literature

Patent Literature 1: JP 2014-8339 A

### Summary

### Technical Problem

In the technique disclosed in Patent Literature 1, however, a plurality of times of input operations is needed in order to perform setting of an operation mode of the ultrasound image and to perform processing in the set operation mode. In order to enhance operability under this situation, it is necessary to reduce the time for which the practitioner stops holding an ultrasound endoscope, and thus, there is a demand for a technique to simplify input operation needed for the operation from operation mode setting to the operation of the mode.

The present invention has been made in view of the foregoing, and an object of the invention is to provide an ultrasound observation system capable of simplifying setting of the operation mode of the ultrasound image and input operation corresponding to the mode.

### Solution to Problem

In order to solve the above described problem and achieve the object, an ultrasound observation system according to the invention includes: an image processing unit configured to generate an ultrasound image based on an ultrasound signal obtained by an ultrasound endoscope configured to transmit ultrasound to a subject as an observation target and receive the ultrasound reflected from the subject; a display unit configured to display the ultrasound image generated by the image processing unit; a multiple input reception unit configured to simultaneously receive inputs at multiple points on the ultrasound image displayed on the display unit; an input information determination unit configured to determine the number of the inputs and positions of the inputs, received by the multiple input reception unit; an operation mode setting unit configured to set an operation mode related to the ultrasound image, based on a result of determination by the input information determination unit; and a target region setting unit configured to set a target region to be processed corresponding to the operation mode set by the operation mode setting unit, based on the positions of the inputs determined by the input information determination unit.

In the ultrasound observation system according to the invention, the operation mode setting unit is configured to set the operation mode based on the result of determination and a display setting mode for the ultrasound image displayed on the display unit.

In the ultrasound observation system according to the invention, the multiple input reception unit is a touch panel provided on a display screen of the display unit.

In the ultrasound observation system according to the invention, the operation mode setting unit is configured to set the operation mode in accordance with the number of the inputs and a positional relationship between the inputs.

The ultrasound observation system according to the invention further includes a control unit configured to detect at least one of the number of times for detection within a predetermined period and duration of detection at a same input position, based on the result of determination by the input information determination unit. The operation mode setting unit is configured to set the operation mode in accordance with a result of detection obtained by the control unit.

In the ultrasound observation system according to the invention, the input information determination unit is configured to further detect a pressure at each of the positions of the inputs. The operation mode setting unit is configured to set the operation mode in accordance with the number of the inputs, each of the positions of the inputs, and the pressure.

### Advantageous Effects of Invention

According to the present invention, it is possible to simplify setting of the operation mode of the ultrasound image and input operation corresponding to the mode.

### Brief Description of Drawings

FIG. 1 is a block diagram illustrating a configuration of an ultrasound observation system equipped with an ultrasound observation apparatus according to a first embodiment of the present invention.
FIG. 2 is a flowchart illustrating processing performed by an operation apparatus of the ultrasound observation system according to the first embodiment of the present invention.
FIG. 3 is a schematic diagram illustrating processing performed by the operation apparatus of the ultrasound observation system according to the first embodiment of the present invention.
FIG. 4 is a schematic diagram illustrating processing performed by the operation apparatus of the ultrasound observation system according to the first embodiment of the present invention.
FIG. 5 is a schematic diagram illustrating processing performed by the operation apparatus of the ultrasound observation system according to the first embodiment of the present invention.
FIG. 6 is a schematic diagram illustrating processing performed by the operation apparatus of the ultrasound observation system according to the first embodiment of the present invention.
FIG. 7 is a schematic diagram illustrating processing performed by the operation apparatus of the ultrasound observation system according to the first embodiment of the present invention.
FIG. 8 is a schematic diagram illustrating processing performed by the operation apparatus of the ultrasound observation system according to the first embodiment of the present invention.
FIG. 9 is a schematic diagram illustrating a display image corresponding to the operation mode set by the operation apparatus of the ultrasound observation system according to the first embodiment of the present invention.
FIG. 10 is a schematic diagram illustrating a display image corresponding to the operation mode set by the operation apparatus of the ultrasound observation system according to the first embodiment of the present invention.
FIG. 11 is a flowchart illustrating processing performed by an operation apparatus of an ultrasound observation system according to a second embodiment of the present invention.
FIG. 12 is a schematic diagram illustrating processing performed by the operation apparatus of the ultrasound observation system according to the second embodiment of the present invention.
FIG. 13 is a schematic diagram illustrating processing performed by the operation apparatus of the ultrasound observation system according to the second embodiment of the present invention.
FIG. 14 is a flowchart illustrating processing performed by an operation apparatus of an ultrasound observation system according to a third embodiment of the present invention.
FIG. 15 is a schematic diagram illustrating processing performed by the operation apparatus of the ultrasound observation system according to the third embodiment of the present invention.
FIG. 16 is a schematic diagram illustrating processing performed by the operation apparatus of the ultrasound observation system according to the third embodiment of the present invention.
FIG. 17 is a flowchart illustrating processing performed by an operation apparatus of an ultrasound observation system according to a fourth embodiment of the present invention.
FIG. 18 is a flowchart illustrating processing performed by an operation apparatus of an ultrasound observation system according to a fifth embodiment of the present invention.
FIG. 19 is a flowchart illustrating processing performed by an operation apparatus of an ultrasound observation system according to a sixth embodiment of the present invention.

### Description of Embodiments

Hereinafter, modes for carrying out the present invention (hereinafter, referred to as embodiment(s)) will be described with reference to the attached drawings. The following description will exemplify an ultrasound observation system for generating an ultrasound image based on an ultrasound echo. The present invention, however, is not limited to these embodiments. The same reference signs are used to designate the same elements throughout the drawings.

### (First Embodiment)

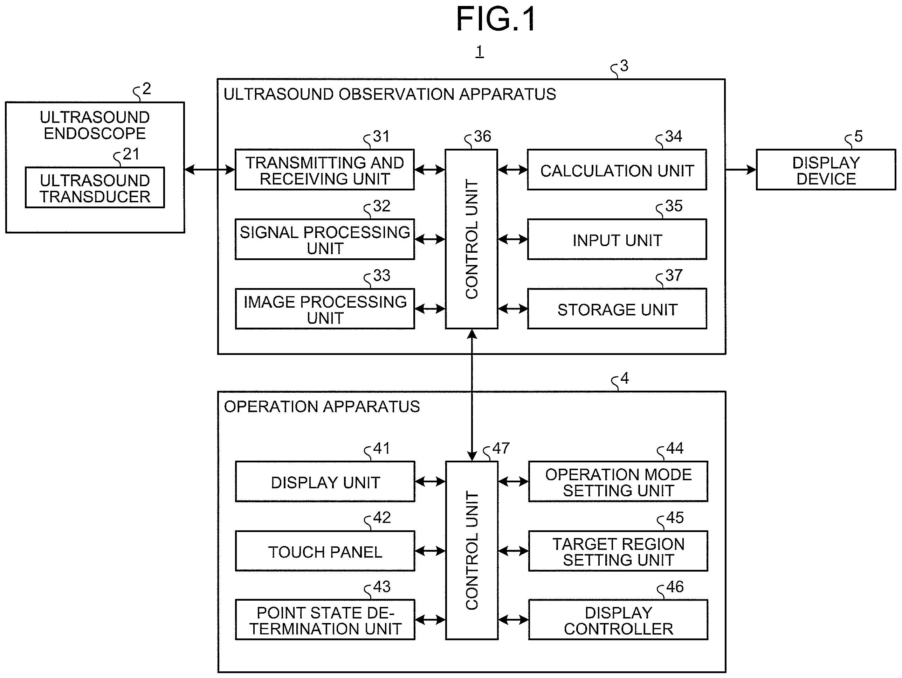

FIG. 1 is a block diagram illustrating a configuration of an ultrasound observation system according to a first embodiment of the present invention. An ultrasound observation system 1 illustrated in the diagram is an apparatus for observing an observation target using ultrasound. The ultrasound observation system 1 includes an ultrasound endoscope 2, an ultrasound observation apparatus 3, an operation apparatus 4, and a display device 5. The ultrasound endoscope 2 transmits ultrasound to a subject as an observation target, and receives ultrasound reflected from the subject. The ultrasound observation apparatus 3 generates an ultrasound image on the basis of an ultrasound signal obtained by the ultrasound endoscope 2. The operation apparatus 4 is capable of simultaneously receiving a plurality of sets of input instruction information, outputs received information to the ultrasound observation apparatus 3, and operates the ultrasound observation apparatus 3. The display device 5 displays the ultrasound image generated by the ultrasound observation apparatus 3.

The ultrasound endoscope 2 includes, on its distal end portion, an ultrasound transducer 21. The ultrasound transducer 21 converts an electrical pulse signal received from the ultrasound observation apparatus 3 into an ultrasound pulse (acoustic pulse) and emits it to the subject. The ultrasound transducer 21 also converts an ultrasound echo reflected from the subject into an electrical echo signal (ultrasound signal) expressed by a voltage change and outputs the signal. The ultrasound transducer 21 may be any of a convex transducer, a linear transducer, and a radial transducer. The ultrasound endoscope 2 may cause the ultrasound transducer 21 to perform mechanical scan, or may provide, as the ultrasound transducer 21, a plurality of elements in an array, and may cause the ultrasound transducer to perform electronic scan by electronically switching elements related to transmission/reception or imposing delay onto transmission/reception of each of elements.

The ultrasound endoscope 2 typically includes imaging optics and imaging elements. The ultrasound endoscope 2 can be inserted into gastrointestinal tracts (esophagus, stomach, duodenum, and large intestine) or respiratory organs (trachea, bronchus) of the subject and can image gastrointestinal tract, respiratory organs, and their surrounding organs (pancreas, gall bladder, bile duct, biliary tract, lymph nodes, mediastinal organs, blood vessels, or the like). The ultrasound endoscope 2 includes a light guide that guides illumination light emitted to the subject at the time of imaging. The light guide is configured such that a distal end portion thereof reaches a distal end of an insertion unit of the ultrasound endoscope 2 into the subject, while a proximal end thereof is connected to a light source device that generates illumination light.

The ultrasound observation apparatus 3 includes a transmitting and receiving unit 31, a signal processing unit 32, an image processing unit 33, a calculation unit 34, an input unit 35, a control unit 36, and a storage unit 37.

The transmitting and receiving unit 31 is electrically connected with the ultrasound endoscope 2, transmits a transmission signal (pulse signal) formed with a high-voltage pulse to the ultrasound transducer 21 on the basis of a predetermined waveform and transmission timing, and together with this, receives an echo signal, that is, an electrical reception signal, from the ultrasound transducer 21, and generates and outputs digital radio frequency (RF) signal data (hereinafter, referred to as RF data).

The frequency band of the pulse signal transmitted by the transmitting and receiving unit 31 is preferably a broadband substantially covering a linear response frequency band for electroacoustic conversion from pulse signals to ultrasound pulses on the ultrasound transducer 21.

The transmitting and receiving unit 31 has a function of transmitting various control signals output by the control unit 36, to the ultrasound endoscope 2, and together with this, has a function of receiving various types of information including identification ID from the ultrasound endoscope 2 and transmitting the information to the control unit 36.

The signal processing unit 32 generates digital reception data for B-mode on the basis of the RF data received from the transmitting and receiving unit 31. Specifically, the signal processing unit 32 performs known processing such as a band-pass filter, envelope detection, logarithmic transformation, on the RF data, and generates digital reception data for B-mode. In logarithmic transformation, a value is represented in decibel value by dividing RF data by the reference voltage V_{c} and then taking a common logarithm of this amount. The signal processing unit 32 outputs the generated reception data for B-mode to the image processing unit 33. The signal processing unit 32 is formed with a central processing unit (CPU), circuits for various types of calculation, or the like.

The image processing unit 33 generates image data on the basis of the RF data received from the transmitting and receiving unit 31. The image processing unit 33 performs signal processing using known techniques, including gain processing and contrast processing, on the RF data, and together with this, generates B-mode image data by performing processing such as data decimation corresponding to a data step size determined in accordance with the display range of the image on the display device 5. The B-mode image is a gray-scale image in which values of R (red), G (green) and B (blue), namely, variables when the RGB color system is employed as a color space, match with each other.

The image processing unit 33 performs coordinate transformation on the reception data for B-mode from the signal processing unit 32 so as to rearrange the scanning range to be correctly represented in space, and thereafter fills gaps among individual reception data for B-mode by performing interpolation processing for individual reception data for B-mode, and generates B-mode image data.

The calculation unit 34 performs calculation processing corresponding to the operation mode to be described below. The calculation unit 34 obtains blood flow information, that is, information related to the flow of blood on the basis of the RF data, for example, and generates a graph and additional information to be superposed onto the B-mode image.

The input unit 35 is formed with an input button that receives input of various types of information such as power on/off.

The control unit 36 controls the entire ultrasound observation system 1. The control unit 36 is formed with a central processing unit (CPU) having calculation and control functions, various calculation circuits, or the like. The control unit 36 reads, from the storage unit 37, information stored in the storage unit 37, and executes various types of calculation processing related to the method for operating the ultrasound observation apparatus 3, thereby integrally controlling the ultrasound observation apparatus 3. The control unit 36 and the signal processing unit 32 can be configured with a common CPU, or the like.

The storage unit 37 stores various programs for operating the ultrasound observation system 1, data including various parameters needed for operation of the ultrasound observation system 1, or the like.

The storage unit 37 also stores various programs including an operation program for executing a method for operating the ultrasound observation system 1. The operation programs can be recorded in a computer-readable recording medium such as a hard disk, flash memory, CD-ROM, DVD-ROM, flexible disk, or the like, and can be distributed broadly. It is also possible to obtain the above-described various programs by downloading them via a communication network. Herein, the communication network refers to one implemented by, for example, a known public network, a local area network (LAN), a wide area network (WAN), regardless of wired or wireless.

The above-configured storage unit 37 is formed with a read only memory (ROM) in which various programs are pre-installed, a random access memory (RAM) that stores calculation parameters and data for each of processing, or the like.

The operation apparatus 4 includes a display unit 41, a touch panel 42 (multiple input reception unit), a point state determination unit 43 (input information determination unit), an operation mode setting unit 44, a target region setting unit 45, a display controller 46, and a control unit 47.

The display unit 41 includes a display panel formed with liquid crystal, organic electro luminescence (EL), or the like. The display unit 41 displays, for example, an ultrasound image that corresponds to the B-mode image data input via the control units 36 and 47, and various types of information regarding operation.

The touch panel 42 is provided on the display screen of the display unit 41 and receives input corresponding to the contact position of an external object. Specifically, the touch panel 42 detects a position touched (contacted) by a user in accordance with the operation icons displayed on the display unit 41 and outputs an operation signal including a position signal corresponding to the detected touch position, onto the control unit 47. The display unit 41 displays the ultrasound image and various types of information, whereby the touch panel 42 functions as a graphical user interface (GUI). The touch panel includes a resistive film type, a capacitive type, an optical type, and any of these touch panels is applicable.

The point state determination unit 43 detects the number of the inputs and a positional relationship between the input positions on the B-mode image on the basis of the operation signal output from the touch panel 42.

The operation mode setting unit 44 sets the operation mode on the basis of the number of the inputs and the positional relationship between the input positions on the B-mode image, detected by the point state determination unit 43. Specifically, the operation mode setting unit 44 sets the mode to any of a pulsed Doppler mode, a flow mode, and an elastography mode.

The pulsed Doppler mode is a mode of analyzing the Doppler shift in a set region (hereinafter, also referred to as a sample volume) and obtaining blood flow information such as the direction of the blood flow. The flow mode is a mode of obtaining blood flow information, that is, information related to the blood flow, by analyzing the Doppler shift in a set region (hereinafter, also referred to as a flow region-of-interest (ROI)) and superposing color information corresponding to the direction of the blood flow, onto the B-mode image. The elastography mode is a mode of obtaining information related to stiffness of an observation target in a set region (hereinafter, also referred to as an elastographic region-of-interest (ROI)) and superposing color information corresponding to the stiffness, onto the B-mode image on the basis of a difference (change amount) between a signal obtained when the ultrasound endoscope 2 is pressed against the observation target and a signal obtained when the ultrasound endoscope 2 is not pressed against the observation target.

The target region setting unit 45 performs region setting processing based on the input position detected by the point state determination unit 43, in accordance with the operation mode set by the operation mode setting unit 44. The target region setting unit 45 performs setting of a one-dimensional region or a two-dimensional region. For example, in a case where the set operation mode is the pulsed Doppler mode, the target region setting unit 45 performs region setting of the sample volume on the basis of the input position. In a case where the set operation mode is the flow mode, the target region setting unit 45 performs region setting for the flow ROI on the basis of the input position. In a case where the set operation mode is the elastography mode, the target region setting unit 45 performs region setting for the elastographic ROI on the basis of the input position.

The display controller 46 performs control of obtaining the B-mode image data generated by the image processing unit 33 and displaying it onto the display unit 41, while performing control of displaying a guidance image of input operation by the touch panel and a display image corresponding to the operation mode, onto the display unit 41.

The control unit 47 controls the overall operation apparatus 4. The control unit 47 is formed with a central processing unit (CPU) having calculation and control functions, various calculation circuits, or the like.

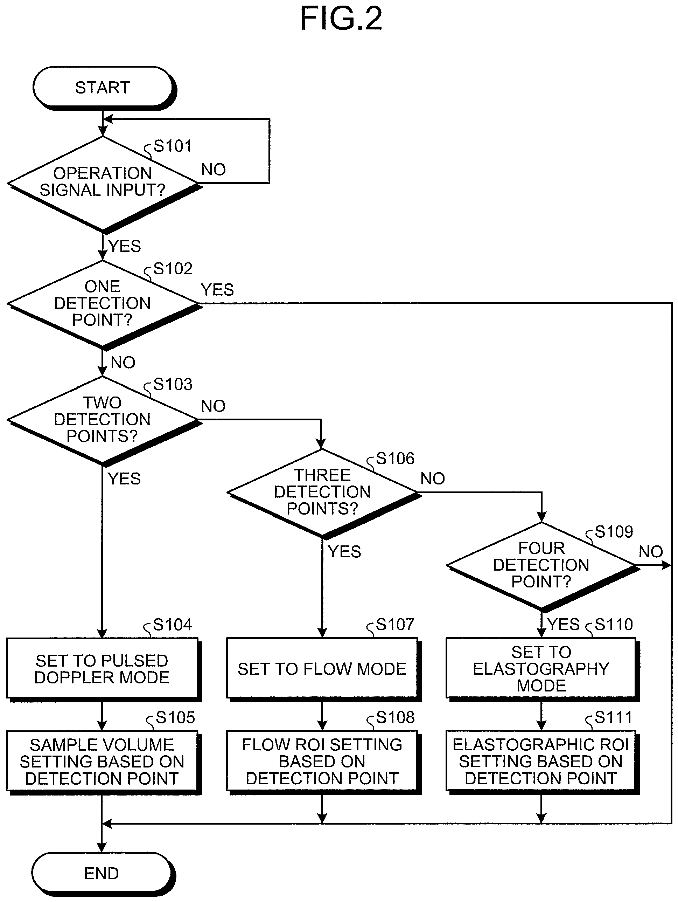

Next, operation mode setting processing performed by the above-configured ultrasound observation apparatus 3 of the ultrasound observation system 1 will be described with reference to the drawings. FIG. 2 is a flowchart illustrating processing performed by the operation apparatus 4 of the ultrasound observation system 1 according to the first embodiment.

First, the control unit 47 judges whether an operation signal including the position signal received by the touch panel 42 is input (step S101). If the operation signal is input (step S101: Yes), the control unit 47 proceeds to step S102. In contrast, if the operation signal is not input (step S101: No), the control unit 47 returns to step S101 and repeats confirmation of input of the operation signal.

In step S102, first, the point state determination unit 43 detects the number of the inputs and the positional relationship between the input positions on the B-mode image on the basis of the position signal detected by the touch panel 42. The control unit 47 obtains the number of inputs detected by the point state determination unit 43 and judges whether the number is one, that is, whether the number of detection points is one.

When the control unit 47 judges that the number of detection points is one (step S102: Yes), the control unit 47 then judges that it is not an operation mode setting target and finishes processing. In contrast, when the control unit 47 judges that the number of detection points is not one, that is, that the number is more than one (step S102: No), the control unit 47 proceeds to step S103. If the number of detection points is one (step S102: Yes), it may be possible to proceed to a processing flow different from the operation mode setting such as normal input button operation and pointer operation.

In step S103, the control unit 47 judges whether the number of detection points is two. Here, when the control unit 47 judges that the number of detection points is two (step S103: Yes), the control unit 47 proceeds to step S104 and performs operation mode setting by the operation mode setting unit 44.

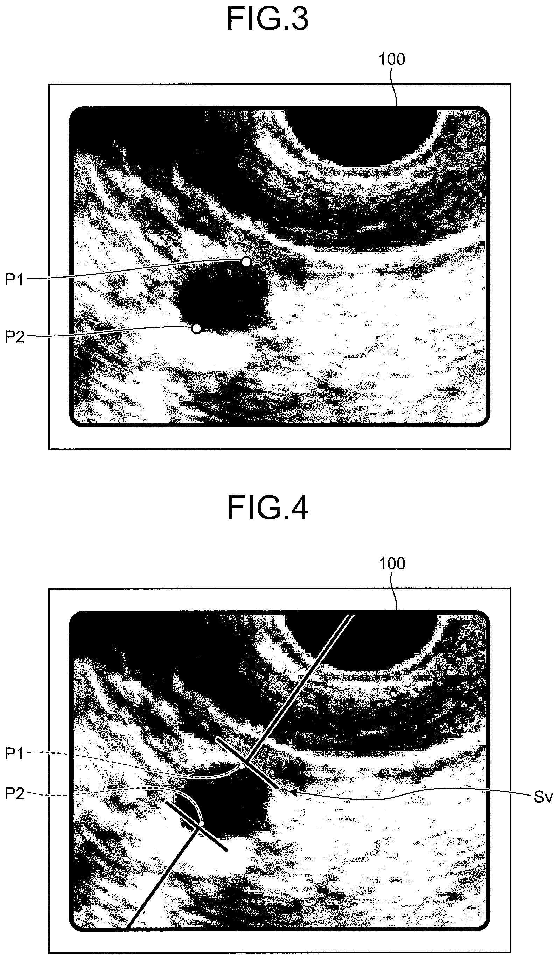

FIG. 3 is a schematic diagram illustrating processing performed by the operation apparatus of the ultrasound observation system according to the first embodiment, in a case where two position signals are input on a B-mode image 100. In step S104, when the operation mode setting unit 44 obtains information that position signals on two points (detection points P1 and P2) have been detected as illustrated in FIG. 3, the operation mode setting unit 44 sets the operation mode to the pulsed Doppler mode. After operation mode setting is performed by the operation mode setting unit 44, the control unit 47 proceeds to step S105.

In step S105, the target region setting unit 45 performs region setting processing based on the input position detected by the point state determination unit 43, in accordance with the operation mode set by the operation mode setting unit 44. Here, since the set operation mode is the pulsed Doppler mode, the target region setting unit 45 performs region setting of the sample volume on the basis of the input position. FIG. 4 is a schematic diagram illustrating processing performed by the operation apparatus of the ultrasound observation system according to the first embodiment, illustrating a sample volume Sv that has been set on the basis of the two position signals on the B-mode image 100. The target region setting unit 45 sets the sample volume Sv having the detection points P1 and P2 as both ends, on the basis of the positional information on the detection points P1 and P2 illustrated in FIG. 3. Thereafter, for example, blood flow information (e.g. graph) in a predetermined region (depth direction) of the set sample volume Sv is generated by the calculation unit 34. In a case where the detection points include a plurality of points (pixels), a centroid position of the region formed by the plurality of points would be used.

In contrast, when the control unit 47 judges in step S103 that the number of detection points is not two (step S103: No), the control unit 47 proceeds to step S106. In step S106, the control unit 47 judges whether the number of detection points is three. Here, when the control unit 47 judges that the number of detection points is three (step S106: Yes), the control unit 47 proceeds to step S107 and performs operation mode setting by the operation mode setting unit 44.

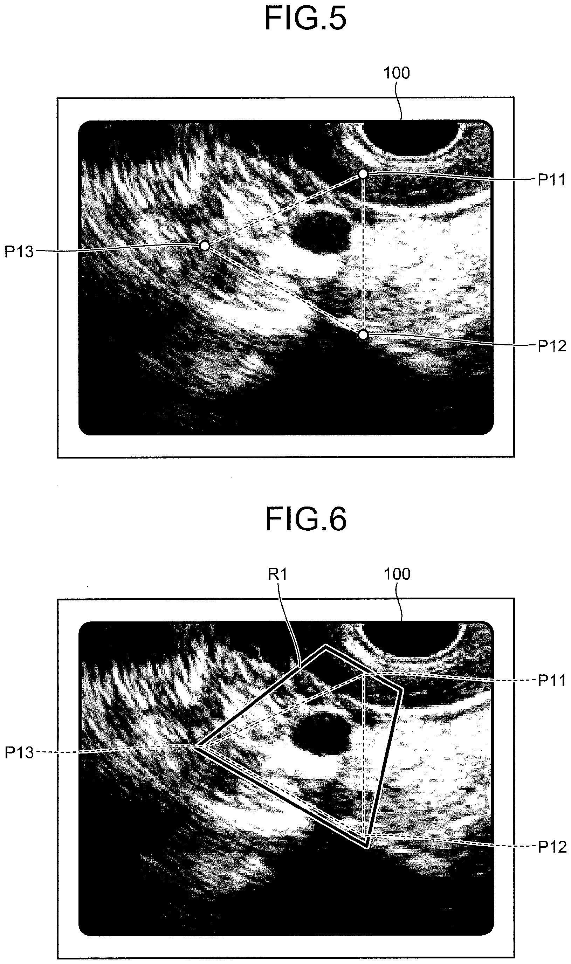

FIG. 5 is a schematic diagram illustrating processing performed by the operation apparatus of the ultrasound observation system according to the first embodiment, in a case where three position signals are input on the B-mode image 100. In step S107, when the operation mode setting unit 44 obtains information that position signals on three points (detection points P11, P12, and P13) have been detected as illustrated in FIG. 5, the operation mode setting unit 44 sets the operation mode to the flow mode. After operation mode setting is performed by the operation mode setting unit 44, the control unit 47 proceeds to step S108.

In step S108, the target region setting unit 45 performs region setting processing based on the input position detected by the point state determination unit 43, in accordance with the operation mode set by the operation mode setting unit 44. Here, since the set operation mode is the flow mode, the target region setting unit 45 performs region setting for the flow region-of-interest on the basis of the input position. FIG. 6 is a schematic diagram illustrating processing performed by the operation apparatus of the ultrasound observation system according to the first embodiment, illustrating a flow region-of-interest R1 (flow ROI) that has been set on the basis of the three position signals on the B-mode image 100. The target region setting unit 45 sets the flow region-of-interest R1 that is a trapezoidal-shaped region inscribing a triangle with each of the detection points P11 to P13 as a vertex, on the basis of the positional information on the detection points P11 to P13 illustrated in FIG. 5. The first embodiment sets, for example, the trapezoidal-shaped flow region-of-interest R1 with the base length at a shallower position in the depth direction being not greater than the base length at a deeper position in the depth direction. Thereafter, for example, the calculation unit 34 generates color information in which red and blue colors are arranged corresponding to the direction of blood flow at the blood vessel position within the flow region-of-interest R1. The trapezoidal-shaped flow region-of-interest R1 is preferably configured such that a line orthogonal to the base of the trapezoid passes through the center or neighborhood of the center of the ultrasound transducer, on the B-mode image.

Moreover, when the control unit 47 judges in step S106 that the number of detection points is not three (step S106: No), the control unit 47 proceeds to step S109. In step S109, the control unit 47 judges whether the number of detection points is four. Here, when the control unit 47 judges that the number of detection points is four (step S109: Yes), the control unit 47 proceeds to step S110 and performs operation mode setting by the operation mode setting unit 44. In contrast, when the control unit 47 judges that the number of detection points is not four (step S109: No), the control unit 47 finishes processing.

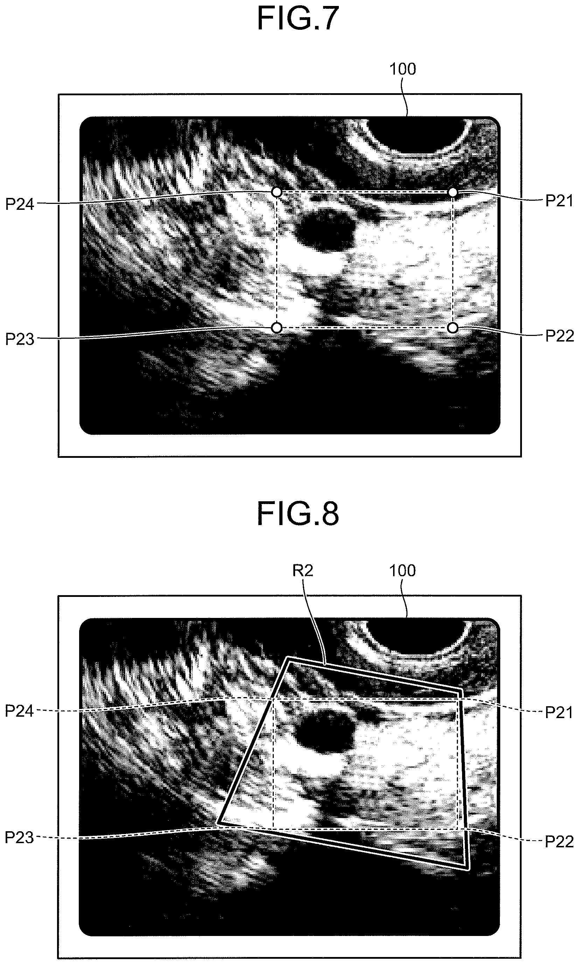

FIG. 7 is a schematic diagram illustrating processing performed by the operation apparatus of the ultrasound observation system according to the first embodiment, in a case where four position signals are input on the B-mode image 100. In step S110, when the operation mode setting unit 44 obtains information that position signals on four points (detection points P21, P22, P23, and P24) have been detected as illustrated in FIG. 7, the operation mode setting unit 44 sets the operation mode to the elastography mode. After operation mode setting is performed by the operation mode setting unit 44, the control unit 47 proceeds to step S111.

In step S111, the target region setting unit 45 performs region setting processing based on the input position detected by the point state determination unit 43, in accordance with the operation mode set by the operation mode setting unit 44. Here, since the set operation mode is the elastography mode, the target region setting unit 45 performs region setting for the elastographic region-of-interest on the basis of the input position. FIG. 8 is a schematic diagram illustrating processing performed by the operation apparatus of the ultrasound observation system according to the first embodiment, illustrating an elastographic region-of-interest R2 (elastographic ROI) that has been set on the basis of four position signals on the B-mode image 100. The target region setting unit 45 sets the elastographic region-of-interest R2 that is a region inscribing at least three points of a square with each of the detection points P21 to P24 as a vertex, on the basis of the positional information on the detection points P21 to P24 illustrated in FIG. 7. Thereafter, for example, color information corresponding to the hardness of the observation target within the set elastographic region-of-interest R2 is generated by the calculation unit 34.

With the processing described above, the practitioner can simultaneously perform setting of the operation mode and setting of the examination region merely by touching (coming in contact with) a plurality of points corresponding to the region determined as an examination target on the B-mode image 100 displayed on the display unit 41. With the setting of the examination region, the calculation unit 34 performs calculation processing corresponding to individual modes. For example, the pulsed Doppler mode is configured to perform calculation processing to achieve waveform display of blood flow (Doppler shift). Moreover, the flow mode is configured to calculate the blood vessel position and blood velocity within the flow region-of-interest R1. The elastography mode is configured to perform calculation processing of the stiffness of the observation target within the elastographic region-of-interest R2.

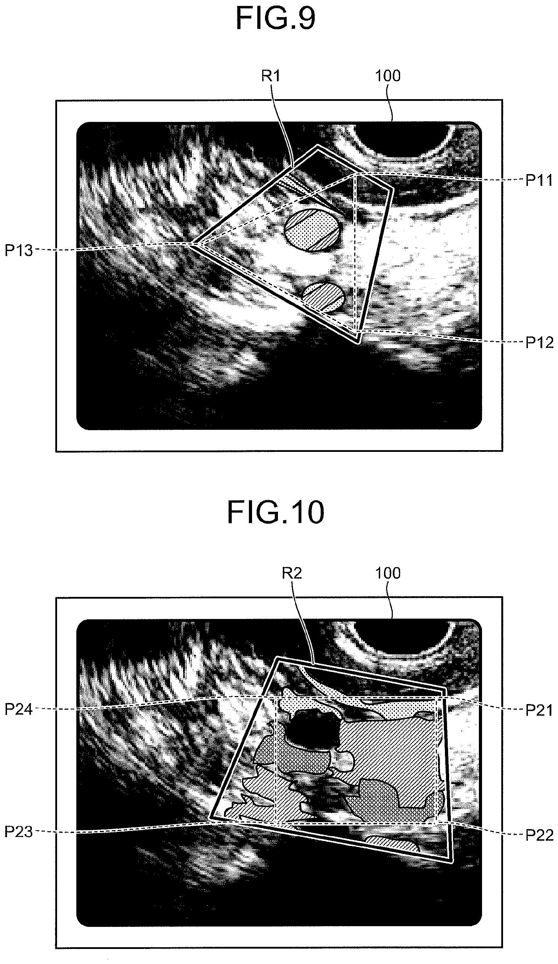

FIG. 9 is a schematic diagram illustrating a display image corresponding to the operation mode set by the operation apparatus of the ultrasound observation system according to the first embodiment of the present invention, illustrating a display image in the flow mode. FIG. 10 is a schematic diagram illustrating a display image corresponding to the operation mode set by the operation apparatus of the ultrasound observation system according to the first embodiment of the present invention, illustrating a display image in the elastography mode.

As illustrated in FIG. 9, the flow mode is configured such that a display image obtained by superposing the blood vessel position within the flow region-of-interest R1 and color information that differs depending on the blood velocity, onto B-mode image, is displayed on the display unit 41 and the display device 5. At this time, the flow region-of-interest R1 and the detection points P11 to P13 may be displayed or not displayed.

Moreover, as illustrated in FIG. 10, the elastography mode is configured such that a display image obtained by superposing color information that differs depending on stiffness of the observation target within the elastographic region-of-interest R2, onto the B-mode image, is displayed on the display unit 41 and the display device 5. At this time, the elastographic region-of-interest R2 and the detection points P21 to P24 may be displayed or not displayed.

With the above-described first embodiment, the point state determination unit 43 is configured to determine the number and position of each of inputs received by the touch panel 42 capable of simultaneously receiving inputs at a plurality of positions on the B-mode image 100 displayed on the display unit 41, the operation mode setting unit 44 is configured to set the operation mode related to the B-mode image 100 on the basis of a result of the determination, and the target region setting unit 45 is configured to set, on the basis of the position of input, a processing target region corresponding to the set operation mode. Accordingly, it is possible to simplify setting of the operation mode of the ultrasound image and input operation corresponding to the mode. Furthermore, it is possible to reduce the time needed for operation.

Moreover, according to the above-described first embodiment, operation input is received by the touch panel 42 capable of simultaneously receiving inputs on a plurality of positions on the B-mode image 100 displayed on the display unit 41. Accordingly, it is possible to perform intuitive operation on the displayed B-mode image.

Moreover, according to the above-described first embodiment, the operation mode is set by a plurality of detection points. Accordingly, it is possible to suppress erroneous setting caused by unintended contact compared with a case where the operation mode is set on the basis of one detection point.

Considering that the practitioner wears gloves in many cases in an operating room, for example, and in a case where the touch panel 42 is operated with a hand with a glove, performing operation mode setting and region setting corresponding to individual operation mode setting with a single touch would be particularly effective in simplification of input operation.

Although two to four detection points are used in the first embodiment, processing may be performed when five detection points are detected. For example, the set operation mode may be canceled when five detection points are detected. Moreover, the operation mode may be changed when touch operation is found after operation mode setting. For example, the mode may proceed to a distance measurement mode (specific processing of the distance measurement mode will be described below) when touch operation is detected while the flow mode is set. If detection of touch operation (detection point) results in error, the practitioner may be notified of retrial of touch operation. Notification methods include display processing by the display unit and processing of outputting light and sound.

In the first embodiment, the sample volume, flow region-of-interest, and the elastographic region-of-interest, set by the target region setting unit 45, may be displayed or not displayed on the B-mode image 100. Moreover, setting of display/not display of the region set by the target region setting unit 45 may be performed by touch operation on the touch panel 42. The name of the set operation mode may be displayed besides the region set by the target region setting unit 45.

Although the pulsed Doppler mode, the flow mode, and the elastography mode are set as the operation modes in the first embodiment, the modes are not limited to these. For example, a contrast harmonic mode or a tissue harmonic mode may be employed. Moreover, arbitrary operation modes may be allocated to the number of detection points, by the practitioner.

### (Second Embodiment)

Next, a second embodiment of the present invention will be described. In the first embodiment, the operation mode is set in accordance with the number of detection points, independently from the display mode of the B-mode image displayed on the display unit 41. In contrast, in the second embodiment, the operation mode is set in accordance with both the display setting mode of the B-mode image displayed on the display unit 41 and the number of detection points.

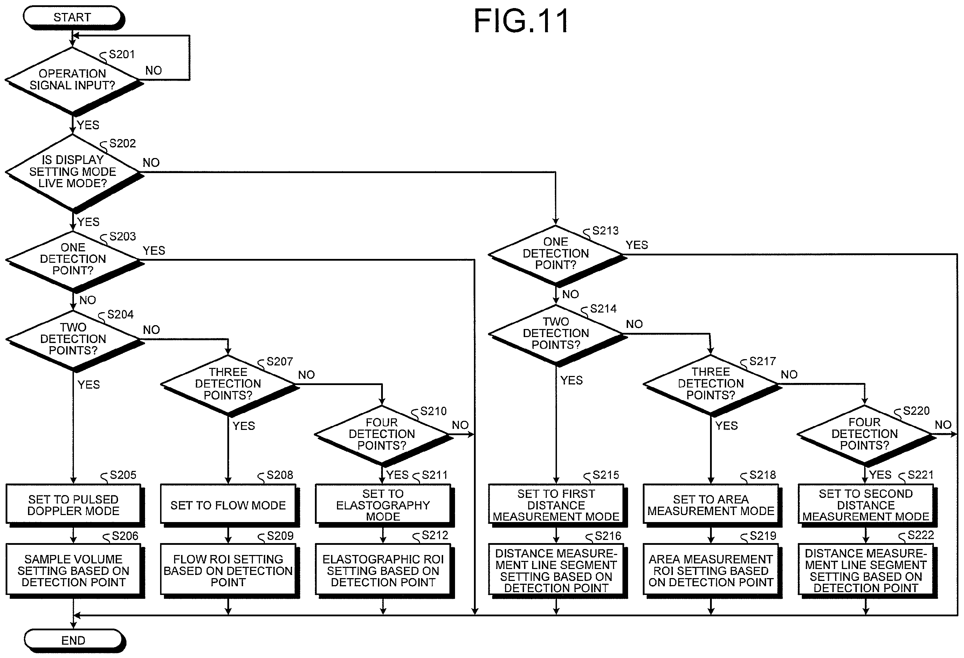

FIG. 11 is a flowchart illustrating processing performed by the operation apparatus 4 of the ultrasound observation system 1 according to the second embodiment. First, the control unit 47 judges whether a position signal received by the touch panel 42 is input (step S201). If the operation signal is input (step S201: Yes), the control unit 47 proceeds to step S202. In contrast, if the operation signal is not input (step S201: No), the control unit 47 returns to step S201 and repeats confirmation of input of the operation signal.

In step S202, the control unit 47 judges whether the display mode of the B-mode image displayed on the display unit 41 is a live mode. Specifically, the control unit 47 judges whether the display setting mode of the B-mode image displayed on the display unit 41 is the live mode or a freeze mode. The live mode is configured to sequentially display B-mode images obtained in time series onto the display unit 41. The freeze mode performs freeze-display of any B-mode image among the generated B-mode images, onto the display unit 41. In a case where the display setting mode of the B-mode image is the live mode (step S202: Yes), the control unit 47 proceeds to step S203.

The display setting mode may be configured such that the mode is switched by touch input into the touch panel 42, or the like, or switched by a signal input via the input unit 35 of the ultrasound observation apparatus 3. The display controller 46 switches display states of the B-mode image of the display unit 41 in accordance with an instruction signal input by these input units. The control unit 47 judges any of which the display setting mode belongs to, with reference to the information on display control performed by the display controller 46.

In step S203 or later steps, operation mode setting and region setting are performed in accordance with the number of detection points (steps S203 to S212), similarly to the above-described steps S102 to S111 in FIG. 2.

In contrast, in a case where the display mode of the B-mode image is not the live mode but the freeze mode (step S202: No), the control unit 47 proceeds to step S213. In step S213, the point state determination unit 43 initially detects the number of the inputs and the positional relationship between the input positions on the B-mode image on the basis of the position signal detected by the touch panel 42. The control unit 47 obtains the number of inputs detected by the point state determination unit 43 and judges whether the number is one, that is, whether the number of detection points is one.

When the control unit 47 judges that the number of detection points is one (step S213: Yes), the control unit 47 then judges that it is not an operation mode setting target and finishes processing. In contrast, when the control unit 47 judges that the number of detection points is not one, that is, that the number is more than one (step S213: No), the control unit 47 proceeds to step S214.

In step S214, the control unit 47 judges whether the number of detection points is two. Here, when the control unit 47 judges that the number of detection points is two (step S214: Yes), the control unit 47 proceeds to step S215 and performs operation mode setting by the operation mode setting unit 44. In step S215, when the operation mode setting unit 44 obtains information that, for example, position signals on two points (detection points P1, and P2) have been detected as illustrated in FIG. 3, the operation mode setting unit 44 sets the operation mode to a first distance measurement mode. After operation mode setting is performed by the operation mode setting unit 44, the control unit 47 proceeds to step S216.

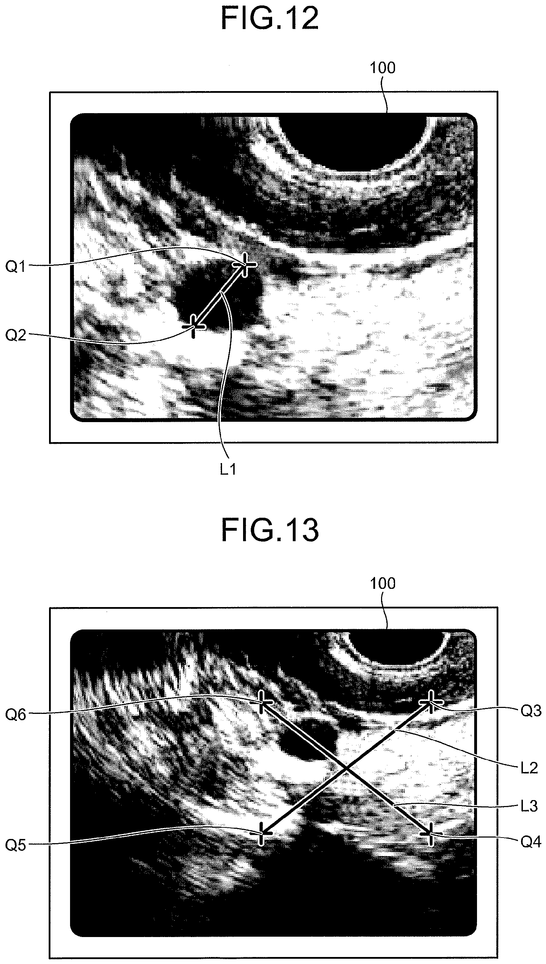

In step S216, the target region setting unit 45 performs region setting processing based on the input position detected by the point state determination unit 43, in accordance with the operation mode set by the operation mode setting unit 44. Here, since the set operation mode is the first distance measurement mode, the target region setting unit 45 performs region setting for a distance measurement line segment on the basis of the input position. FIG. 12 is a schematic diagram illustrating processing performed by the operation apparatus of the ultrasound observation system according to the second embodiment, illustrating a distance measurement line segment L1 that has been set on the basis of the two position signals on the B-mode image 100. The target region setting unit 45 sets the distance measurement line segment L1, that is, a line segment connecting centers Q1 and Q2 (refer to FIG. 12) of the detection points P1 and P2 with each other, for example, on the basis of positional information on the detection points P1 and P2, illustrated in FIG. 3, for example. Thereafter, for example, the length corresponding to the set distance measurement line segment L1 is measured and the distance on an actual observation target is calculated on the basis of magnification of the B-mode image, or the like, by the calculation unit 34.

In contrast, when the control unit 47 judges in step S214 that the number of detection points is not two (step S214: No), the control unit 47 proceeds to step S217. In step S217, the control unit 47 judges whether the number of detection points is three. Here, when the control unit 47 judges that the number of detection points is three (step S217: Yes), the control unit 47 proceeds to step S218 and performs operation mode setting by the operation mode setting unit 44.

In step S218, when the operation mode setting unit 44 obtains information that, for example, position signals on three points (detection points P11, P12, and P13) have been detected as illustrated in FIG. 5, the operation mode setting unit 44 sets the operation mode to an area measurement mode. After operation mode setting is performed by the operation mode setting unit 44, the control unit 47 proceeds to step S219.

In step S219, the target region setting unit 45 performs region setting processing based on the input position detected by the point state determination unit 43, in accordance with the operation mode set by the operation mode setting unit 44. Here, since the set operation mode is the area measurement mode, the target region setting unit 45 performs region setting for an area measurement region-of-interest (ROI) on the basis of the input position. The target region setting unit 45 sets the area measurement region-of-interest that is a circle inscribing a triangle with each of the detection points P11 to P13 as a vertex, on the basis of the positional information on the detection points P11 to P13 illustrated in FIG. 5. Thereafter, for example, the area of the set circle is measured and the area on an actual observation target is calculated on the basis of magnification of the B-mode image, or the like, by the calculation unit 34.

Moreover, when the control unit 47 judges in step S217 that the number of detection points is not three (step S217: No), the control unit 47 proceeds to step S220. In step S220, the control unit 47 judges whether the number of detection points is four. Here, when the control unit 47 judges that the number of detection points is four (step S220: Yes), the control unit 47 proceeds to step S221 and performs operation mode setting by the operation mode setting unit 44. In contrast, when the control unit 47 judges that the number of detection points is not four (step S220: No), the control unit 47 finishes processing.

In step S221, when the operation mode setting unit 44 obtains information that, for example, position signals on four points (detection points P21, P22, P23, and P24) have been detected as illustrated in FIG. 7, the operation mode setting unit 44 sets the operation mode to a second distance measurement mode. After operation mode setting is performed by the operation mode setting unit 44, the control unit 47 proceeds to step S222.

In step S222, the target region setting unit 45 performs region setting processing based on the input position detected by the point state determination unit 43, in accordance with the operation mode set by the operation mode setting unit 44. Here, since the set operation mode is the second distance measurement mode, the target region setting unit 45 performs region setting corresponding to a distance measurement line segment on the basis of the input position. FIG. 13 is a schematic diagram illustrating processing performed by the operation apparatus of the ultrasound observation system according to the second embodiment. On the basis of the positional information on the detection points P21 to P24 illustrated in FIG. 7, the target region setting unit 45 sets two distance measurement line segments L2 and L3, which are two line segments connecting individual centers (center Q3 to center Q5, and center Q4 to center Q6) of opposing detection points (for example, diagonally positioned detection points P21 with P23, and P22 with P24). Thereafter, for example, the length corresponding to each of the two distance measurement line segments L2 and L3 that have been set are measured and the distance on an actual observation target is calculated on the basis of magnification of the B-mode image, or the like, by the calculation unit 34.

With the processing described above, the practitioner can simultaneously perform setting of the operation mode and setting of the examination region merely by touching (coming in contact with) a plurality of points corresponding to the region determined as an examination target on the B-mode image 100 displayed on the display unit 41. Furthermore, the second embodiment judges which mode the display setting mode of the display unit 41 belongs to, and sets the operation mode corresponding to the display mode. Therefore, it is possible to select and set the operation mode corresponding to the display state being examined, with a single touch operation. Moreover, by displaying the image information generated in accordance with individual operation modes and displaying measurement information on the display unit 41 and the display device 5, it is possible to perform operation from operation mode setting to processing result display with individual operation modes, with a single touch operation.

With the above-described second embodiment, the point state determination unit 43 is configured to determine the number and position of each of inputs received by the touch panel 42 capable of simultaneously receiving inputs at a plurality of positions on the B-mode image 100 displayed on the display unit 41, the operation mode setting unit 44 is configured to set the operation mode related to the B-mode image 100 on the basis of a result of the determination and the display setting mode of the display unit 41, and the target region setting unit 45 is configured to set, on the basis of the position of input, a processing target region corresponding to the set operation mode. Accordingly, it is possible to simplify setting of the operation mode of the ultrasound image and input operation corresponding to the mode.

Although the decision of step S202 is made in accordance with the display setting mode in the flowchart illustrated in FIG. 11 according to the second embodiment, the decision may be made in accordance with the current operation mode. For example, if the current operation mode is a mode for displaying the B-mode image, the processing may proceed to step S203, and if the current operation mode is a contrast harmonic mode, the processing may proceed to step S213.

### (Third Embodiment)

Next, a third embodiment of the present invention will be described. While the first embodiment assumes that the pulsed Doppler mode is set in a case where the number of detection points is two, the third embodiment sets the operation modes that differ depending on the direction of the line segment connecting the detection points with each other, even in a case where the number of detection points is two.

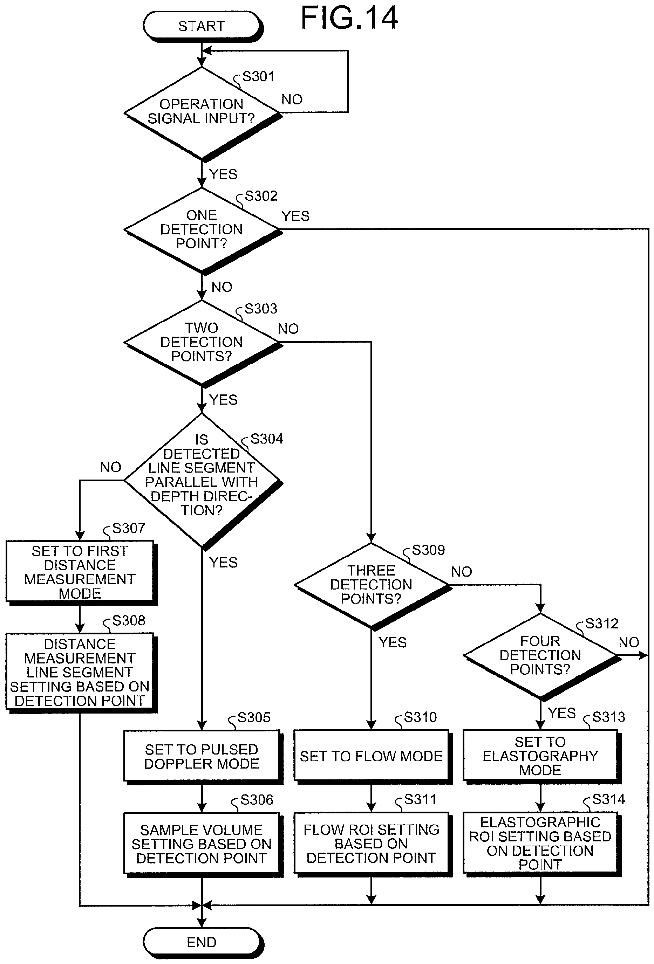

FIG. 14 is a flowchart illustrating processing performed by the operation apparatus 4 of the ultrasound observation system 1 according to the third embodiment. First, the control unit 47 judges whether a position signal received by the touch panel 42 is input (step S301). If the operation signal is input (step S301: Yes), the control unit 47 proceeds to step S302. In contrast, if the operation signal is not input (step S301: No), the control unit 47 returns to step S301 and repeats confirmation of input of the operation signal.

In step S302, the point state determination unit 43 initially detects the number of the inputs and the positional relationship between the input positions on the B-mode image on the basis of the position signal detected by the touch panel 42. The control unit 47 obtains the number of inputs detected by the point state determination unit 43 and judges whether the number is one, that is, whether the number of detection points is one.

When the control unit 47 judges that the number of detection points is one (step S302: Yes), the control unit 47 then judges that it is not an operation mode setting target and finishes processing. In contrast, when the control unit 47 judges that the number of detection points is not one, that is, that the number is more than one (step S302: No), the control unit 47 proceeds to step S303.

In step S303, the control unit 47 judges whether the number of detection points is two. Here, when the control unit 47 judges that the number of detection points is two (step S303: Yes), the control unit 47 proceeds to step S304.

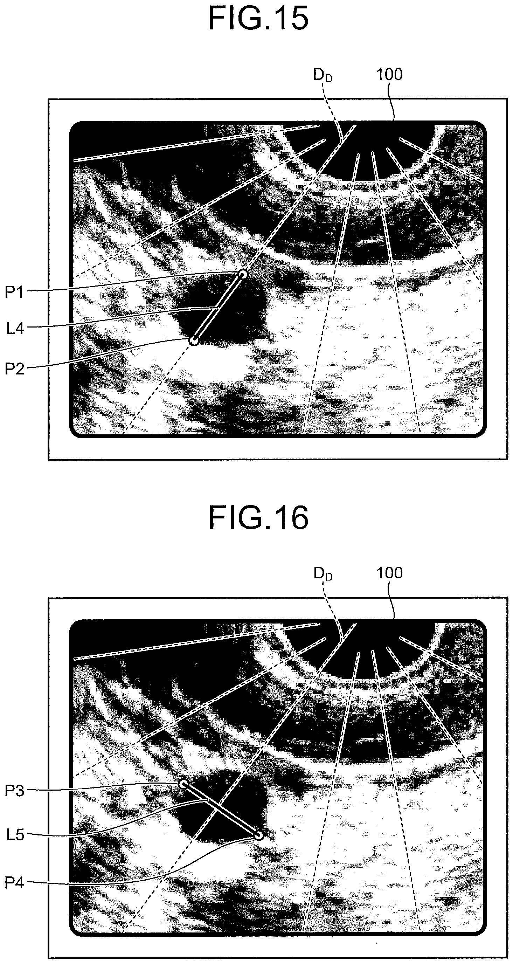

In step S304, the control unit 47 judges whether the direction of a line segment connecting the detection points with each other is parallel with the depth direction of the observation target (direction (sound ray direction) orthogonal to the arrangement direction of elements of the ultrasound transducer 21). FIG. 15 is a schematic diagram illustrating processing performed by the operation apparatus of the ultrasound observation system according to the third embodiment, illustrating an exemplary case where the line segment connecting the detected two points is parallel with the depth direction. As illustrated in FIG. 15, in a case where a line segment L4 connecting centers of the two detected points (detection points P1 and P2) with each other is parallel with a depth direction D_{D}, (step S304: Yes), the control unit 47 proceeds to step S305. In step S304, the control unit 47 judges whether the line segment is parallel with the depth direction by judging, for example, whether two points exist on a same line among the lines in the depth direction, or, whether a line extending along the line segment connecting the two points with each other passes through a center position of the transducer.

In step S305, the operation mode setting unit 44 sets the operation mode to the pulsed Doppler mode. After operation mode setting is performed by the operation mode setting unit 44, the control unit 47 proceeds to step S306. As described above, in step S306, the target region setting unit 45 performs region setting of the sample volume on the basis of the input position.

In contrast, in a case where the control unit 47 judges that the direction of a line segment connecting the detection points with each other is not parallel with the depth direction of the observation target (step S304: No), the control unit 47 proceeds to step S307. FIG. 16 is a schematic diagram illustrating processing performed by the operation apparatus of the ultrasound observation system according to the third embodiment, illustrating an exemplary case where the line segment connecting the detected two points with each other is not parallel with the depth direction. As illustrated in FIG. 16, in a case where a line segment L5 connecting centers of the two detected points (detection points P3 and P4) with each other is not parallel with (e.g. intersecting with) a depth direction D_{D}, the control unit 47 proceeds to step S307.

In step S307, the operation mode setting unit 44 sets the operation mode to the first distance measurement mode. After operation mode setting is performed by the operation mode setting unit 44, the control unit 47 proceeds to step S308. In step S308, the target region setting unit 45 sets the distance measurement line segment L5, that is, a line segment connecting the detection points P3 and P4 with each other.

In contrast, when the control unit 47 judges in step S303 that the number of detection points is not two (step S303: No), the control unit 47 proceeds to step S309. In step S309, the control unit 47 judges whether the number of detection points is three. Here, when the control unit 47 judges that the number of detection points is three (step S309: Yes), the control unit 47 proceeds to step S310 and performs operation mode setting by the operation mode setting unit 44.

In step S310, the operation mode setting unit 44 sets the operation mode to the flow mode. After operation mode setting is performed by the operation mode setting unit 44, the control unit 47 proceeds to step S311. In step S311, the target region setting unit 45 performs region setting of the flow region-of-interest on the basis of the input position.

Moreover, when the control unit 47 judges in step S309 that the number of detection points is not three (step S309: No), the control unit 47 proceeds to step S312. In step S312, the control unit 47 judges whether the number of detection points is four. Here, when the control unit 47 judges that the number of detection points is four (step S312: Yes), the control unit 47 proceeds to step S313 and performs operation mode setting by the operation mode setting unit 44. In contrast, when the control unit 47 judges that the number of detection points is not four (step S312: No), the control unit 47 finishes processing.

In step S313, the operation mode setting unit 44 sets the operation mode to the elastography mode. After operation mode setting is performed by the operation mode setting unit 44, the control unit 47 proceeds to step S314. In step S314, the target region setting unit 45 performs region setting of the elastographic region-of-interest on the basis of the input position.

With the processing described above, the practitioner can simultaneously perform setting of the operation mode and setting of the examination region merely by touching (coming in contact with) a plurality of points corresponding to the region determined as an examination target on the B-mode image 100 displayed on the display unit 41. In the third embodiment, in a case where the number of detection points is two, the mode is set to any of the pulsed Doppler mode and the first distance measurement mode depending on the positional relationship between the detection points, thereby enabling further specific setting of the operation mode by a single touch operation.

With the above-described third embodiment, the point state determination unit 43 is configured to determine the number and position of each of inputs (detection points) received by the touch panel 42 capable of simultaneously receiving inputs at a plurality of positions on the B-mode image 100 displayed on the display unit 41, the operation mode setting unit 44 is configured to set the operation mode related to the B-mode image 100 on the basis of a result of the determination including the positional relationship between the detection points and on the basis of the display setting mode of the display unit 41, and the target region setting unit 45 is configured to set, on the basis of the position of input, a processing target region corresponding to the set operation mode. Accordingly, it is possible to simplify setting of the operation mode of the ultrasound image and input operation corresponding to the mode.

Although the number of detection points is two in the third embodiment, four detection points may be employed to compare the line segment connecting designated two points with the depth direction to set either the elastography mode or the second distance measurement mode.

### (Fourth Embodiment)

Next, a fourth embodiment of the present invention will be described. The first embodiment assumes that the flow mode is set in a case where the number of detection points is three. In the fourth embodiment, however, the mode is switched to the flow mode with different color information setting when a plurality of times of touch operations is detected within a predetermined period even when the number of detection points is three.

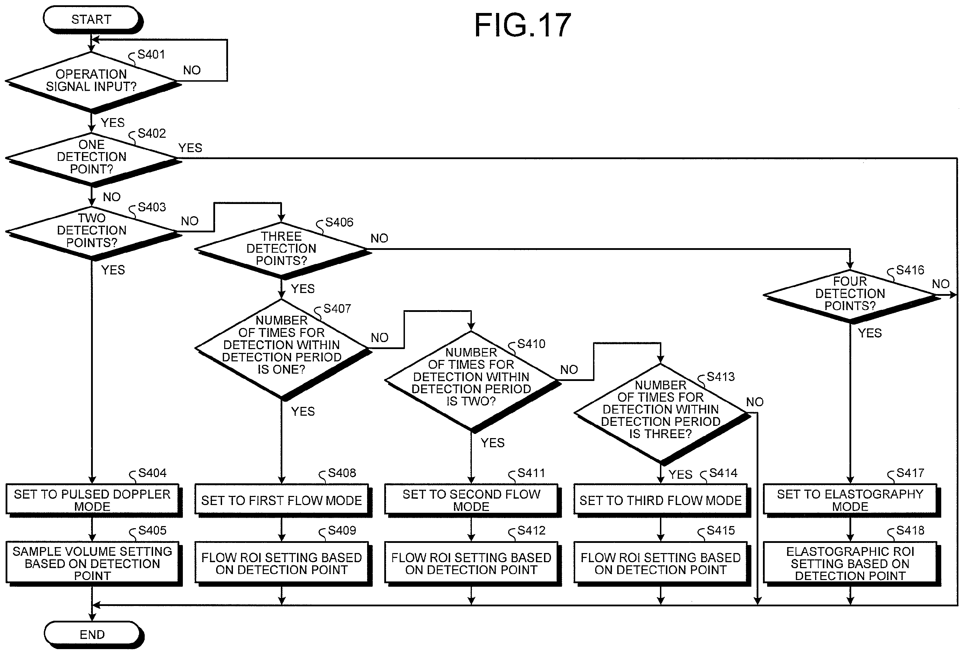

FIG. 17 is a flowchart illustrating processing performed by the operation apparatus 4 of the ultrasound observation system 1 according to the fourth embodiment. First, the control unit 47 judges whether a position signal received by the touch panel 42 is input (step S401). If the operation signal is input (step S401: Yes), the control unit 47 proceeds to step S402. In contrast, if the operation signal is not input (step S401: No), the control unit 47 returns to step S401 and repeats confirmation of input of the operation signal.

In step S402, the point state determination unit 43 initially detects the number of the inputs and a positional relationship between the input positions on the B-mode image on the basis of the position signal detected by the touch panel 42. The control unit 47 obtains the number of inputs detected by the point state determination unit 43 and judges whether the number is one, that is, whether the number of detection points is one.

When the control unit 47 judges that the number of detection points is one (step S402: Yes), the control unit 47 then judges that it is not an operation mode setting target and finishes processing. In contrast, when the control unit 47 judges that the number of detection points is not one, that is, when the number is more than one (step S402: No), the control unit 47 proceeds to step S403.

In step S403, the control unit 47 judges whether the number of detection points is two. Here, when the control unit 47 judges that the number of detection points is two (step S403: Yes), the control unit 47 proceeds to step S404.

In step S404, the operation mode setting unit 44 sets the operation mode to the pulsed Doppler mode. After operation mode setting is performed by the operation mode setting unit 44, the control unit 47 proceeds to step S405. As described above, in step S405, the target region setting unit 45 performs region setting of the sample volume on the basis of the input position.

In contrast, when the control unit 47 judges in step S403 that the number of detection points is not two (step S403: No), the control unit 47 proceeds to step S406. In step S406, the control unit 47 judges whether the number of detection points is three. Here, when the control unit 47 judges that the number of detection points is three (step S406: Yes), the control unit 47 proceeds to step S407.

In step S407, the control unit 47 judges whether the number of inputs of operation signals for the three points within a predetermined period (number of times for detection within the predetermined period) is one. When the control unit 47 judges that the number of inputs of the operation signals for the three points within the predetermined period is one (step S407: Yes), the control unit 47 proceeds to step S408 and performs operation mode setting by the operation mode setting unit 44. In step S408, the operation mode setting unit 44 sets the operation mode to a first flow mode. After operation mode setting is performed by the operation mode setting unit 44, the control unit 47 proceeds to step S409. In step S409, the target region setting unit 45 performs region setting of the flow region-of-interest on the basis of the input position.

In step S409, for example, the target region setting unit 45 sets the flow region-of-interest R1 as illustrated in FIG. 6. Thereafter, for example, color information corresponding to the blood velocity within the set flow region-of-interest R1 is generated by the calculation unit 34. The first flow mode is configured, for example, to generate color information in which red and blue colors are arranged according to the blood flow direction.

Moreover, when the control unit 47 judges in step S407 that the number of inputs of the operation signals for the three detection points within a predetermined period is not one (step S407: No), the control unit 47 proceeds to step S410. In step S410, the control unit 47 judges whether the number of inputs of operation signals for the three points within a predetermined period is two. When the control unit 47 judges that the number of inputs of the operation signals for the three points within the predetermined period is two (step S410: Yes), the control unit 47 proceeds to step S411 and performs operation mode setting by the operation mode setting unit 44.

In step S411, the operation mode setting unit 44 sets the operation mode to a second flow mode. After operation mode setting is performed by the operation mode setting unit 44, the control unit 47 proceeds to step S412. In step S412, the target region setting unit 45 performs region setting of the flow region-of-interest on the basis of the input position.

In step S412, for example, the target region setting unit 45 sets the flow region-of-interest R1 as illustrated in FIG. 6. Thereafter, for example, color information corresponding to the blood velocity within the set flow region-of-interest R1 is generated by the calculation unit 34. The second flow mode is configured, for example, to generate color information in which green and orange colors are arranged according to the blood flow direction.

Moreover, when the control unit 47 judges in step S410 that the number of inputs of the operation signals for the three detection points within a predetermined period is not two (step S410: No), the control unit 47 proceeds to step S413. In step S413, the control unit 47 judges whether the number of inputs of operation signals for the three points within a predetermined period is three. When the control unit 47 judges that the number of inputs of the operation signals for the three points within the predetermined period is three (step S413: Yes), the control unit 47 proceeds to step S414 and performs operation mode setting by the operation mode setting unit 44. Moreover, when the control unit 47 judges that the number of inputs of the operation signals for the three detection points within a predetermined period is not three, that is, four or more (step S413: No), the control unit 47 finishes processing.

In step S414, the operation mode setting unit 44 sets the operation mode to a third flow mode. After operation mode setting is performed by the operation mode setting unit 44, the control unit 47 proceeds to step S415. In step S415, the target region setting unit 45 performs region setting of the flow region-of-interest on the basis of the input position.

In step S415, for example, the target region setting unit 45 sets the flow region-of-interest R1 as illustrated in FIG. 6. Thereafter, for example, color information corresponding to the blood velocity within the set flow region-of-interest R1 is generated by the calculation unit 34. The third flow mode is configured, for example, to generate color information in which orange shading is changed according to the presence or absence of blood flow. The mode that displays the presence or absence of blood flow using color information, as the third flow mode, is also referred to as a power flow mode.

In contrast, when the control unit 47 judges in step S406 that the number of detection points is not three (step S406: No), the control unit 47 proceeds to step S416. In step S416, the control unit 47 judges whether the number of detection points is four. Here, when the control unit 47 judges that the number of detection points is four (step S416: Yes), the control unit 47 proceeds to step S417 and performs operation mode setting by the operation mode setting unit 44. In contrast, when the control unit 47 judges that the number of detection points is not four (step S416: No), the control unit 47 finishes processing.

In step S417, the operation mode setting unit 44 sets the operation mode to the elastography mode. After operation mode setting is performed by the operation mode setting unit 44, the control unit 47 proceeds to step S418. In step S418, the target region setting unit 45 performs region setting of the elastographic region-of-interest on the basis of the input position.

With the processing described above, the practitioner can simultaneously perform setting of the operation mode and setting of the examination region merely by touching (coming in contact with) a plurality of points corresponding to the region determined as an examination target on the B-mode image 100 displayed on the display unit 41. In the fourth embodiment, in a case where the number of detection points is three, the mode is set to a different flow mode corresponding to the number of times for touching of the detection points, thereby enabling further specific setting of the operation mode by a single touch operation.

With the above-described fourth embodiment, the point state determination unit 43 is configured to determine the number and position of each of inputs received by the touch panel 42 capable of simultaneously receiving inputs at a plurality of positions on the B-mode image 100 displayed on the display unit 41, the operation mode setting unit 44 is configured to set the operation mode related to the B-mode image 100 on the basis of a result of the determination including the number of times for touching of the detection points, and the target region setting unit 45 is configured to set, on the basis of the position of input, a processing target region corresponding to the set operation mode. Accordingly, it is possible to simplify setting of the operation mode of the ultrasound image and input operation corresponding to the mode.

### (Fifth Embodiment)

Next, a fifth embodiment of the present invention will be described. The first embodiment assumes that the flow mode is set in a case where the number of detection points is three. In the fifth embodiment, however, the mode is switched to the flow mode with different color information setting corresponding to a maintenance period of a contact state even when the number of detection points is three.

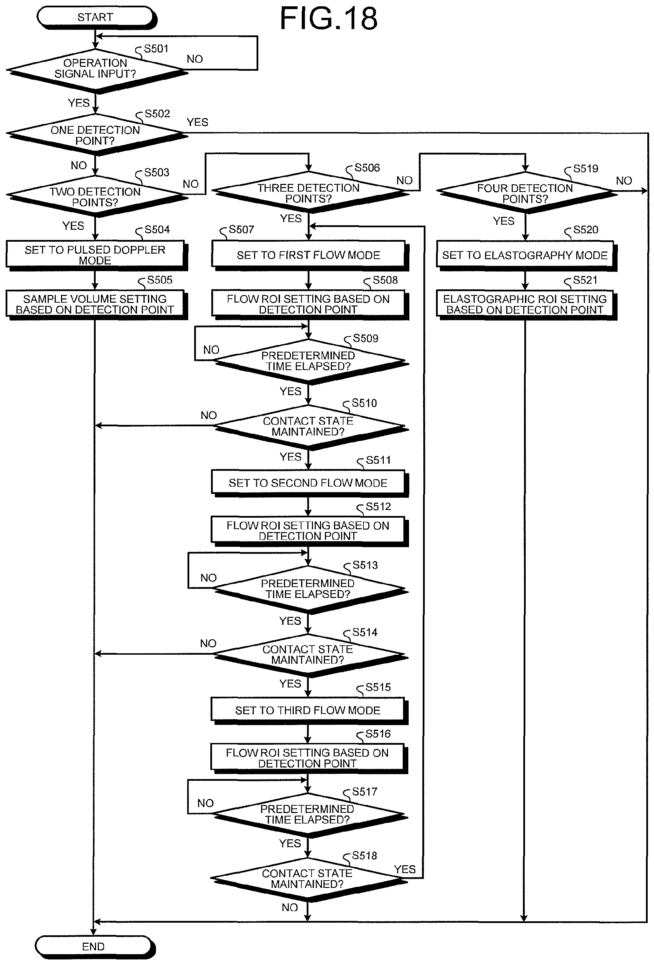

FIG. 18 is a flowchart illustrating processing performed by the operation apparatus 4 of the ultrasound observation system 1 according to the fifth embodiment. First, the control unit 47 judges whether a position signal received by the touch panel 42 is input (step S501). If the operation signal is input (step S501: Yes), the control unit 47 proceeds to step S502. In contrast, if the operation signal is not input (step S501: No), the control unit 47 returns to step S501 and repeats confirmation of input of the operation signal.

In step S502, first, the point state determination unit 43 detects the number of the inputs and a positional relationship between the input positions on the B-mode image on the basis of the position signal detected by the touch panel 42. The control unit 47 obtains the number of inputs detected by the point state determination unit 43 and judges whether the number is one, that is, whether the number of detection points is one.

When the control unit 47 judges that the number of detection points is one (step S502: Yes), the control unit 47 then judges that it is not an operation mode setting target and finishes processing. In contrast, when the control unit 47 judges that the number of detection points is not one, that is, that the number is more than one (step S502: No), the control unit 47 proceeds to step S503.

In step S503, the control unit 47 judges whether the number of detection points is two. Here, when the control unit 47 judges that the number of detection points is two (step S503: Yes), the control unit 47 proceeds to step S504.

In step S504, the operation mode setting unit 44 sets the operation mode to the pulsed Doppler mode. After operation mode setting is performed by the operation mode setting unit 44, the control unit 47 proceeds to step S505. As described above, in step S505, the target region setting unit 45 performs region setting of the sample volume on the basis of the input position.

In contrast, when the control unit 47 judges in step S503 that the number of detection points is not two (step S503: No), the control unit 47 proceeds to step S506. In step S506, the control unit 47 judges whether the number of detection points is three. Here, when the control unit 47 judges that the number of detection points is three (step S506: Yes), the control unit 47 proceeds to step S507.

In step S507, operation mode setting is performed by the operation mode setting unit 44. In step S507, the operation mode setting unit 44 sets the operation mode to the first flow mode. After operation mode setting is performed by the operation mode setting unit 44, the control unit 47 proceeds to step S508. In step S508, the target region setting unit 45 performs region setting of the flow region-of-interest on the basis of the input position.

In step S508, as described above, for example, the target region setting unit 45 sets the flow region-of-interest R1 as illustrated in FIG. 6. Thereafter, for example, color information corresponding to the blood flow direction within the set flow region-of-interest R1 is generated by the calculation unit 34. The first flow mode is configured, for example, to generate color information in which red and blue colors are arranged according to the blood flow direction. The color information generated by the calculation unit 34 is superposed onto the B-mode image and displayed on the display unit 41 or the display device 5.

Thereafter, the control unit 47 judges whether a predetermined period of time (e.g. 0.5 second) has elapsed after input of an operation signal (step S509). Here, when the control unit 47 judges that the predetermined period of time has not elapsed (step S509: No), the control unit 47 returns to step S509 and repeats judgment of passage of time. In contrast, in a case where the control unit 47 judges that the predetermined period of time has elapsed (step S509: Yes), the control unit 47 proceeds to step S510. In step S510, the control unit 47 judges whether the contact with the touch panel 42 according to the input operation signal is continued, in other words, whether the contact state with the touch panel 42 is maintained. When the control unit 47 judges that the contact state with the touch panel 42 is maintained (step S510: Yes), the control unit 47 proceeds to step S511 and performs operation mode setting by the operation mode setting unit 44. In contrast, when the control unit 47 judges that the contact state with the touch panel 42 is not maintained (step S510: No), the control unit 47 finishes processing.

In step S511, the operation mode setting unit 44 sets the operation mode to the second flow mode. After operation mode setting is performed by the operation mode setting unit 44, the control unit 47 proceeds to step S512. In step S512, the target region setting unit 45 performs region setting of the flow region-of-interest on the basis of the input position.

Thereafter, the control unit 47 judges whether a predetermined period of time has elapsed after input of an operation signal (step S513). Here, when the control unit 47 judges that the predetermined period of time has not elapsed (step S513: No), the control unit 47 returns to step S513 and repeats judgment of passage of time. In contrast, in a case where the control unit 47 judges that the predetermined period of time has elapsed (step S513: Yes), the control unit 47 proceeds to step S514. In step S514, the control unit 47 judges whether the contact state with the touch panel 42 is maintained. When the control unit 47 judges that the contact state with the touch panel 42 is maintained (step S514: Yes), the control unit 47 proceeds to step S515 and performs operation mode setting by the operation mode setting unit 44. In contrast, when the control unit 47 judges that the contact state with the touch panel 42 is not maintained (step S514: No), the control unit 47 finishes processing.

In step S515, the operation mode setting unit 44 sets the operation mode to the third flow mode. After operation mode setting is performed by the operation mode setting unit 44, the control unit 47 proceeds to step S516. In step S516, the target region setting unit 45 performs region setting of the flow region-of-interest on the basis of the input position.

Thereafter, the control unit 47 judges whether a predetermined period of time has elapsed after input of an operation signal (step S517). Here, when the control unit 47 judges that the predetermined period of time has not elapsed (step S517: No), the control unit 47 returns to step S517 and repeats judgment of passage of time. In contrast, in a case where the control unit 47 judges that the predetermined period of time has elapsed (step S517: Yes), the control unit 47 proceeds to step S518. In step S518, the control unit 47 judges whether the contact state with the touch panel 42 is maintained. When the control unit 47 judges that the contact state with the touch panel 42 is maintained (step S518: Yes), the control unit 47 proceeds to step S507 and performs operation mode setting by the operation mode setting unit 44. In contrast, when the control unit 47 judges that the contact state with the touch panel 42 is not maintained (step S518: No), the control unit 47 finishes processing.

In contrast, when the control unit 47 judges in step S506 that the number of detection points is not three (step S506: No), the control unit 47 proceeds to step S519. In step S519, the control unit 47 judges whether the number of detection points is four. Here, when the control unit 47 judges that the number of detection points is four (step S519: Yes), the control unit 47 proceeds to step S520 and performs operation mode setting by the operation mode setting unit 44. In contrast, when the control unit 47 judges that the number of detection points is not four (step S519: No), the control unit 47 finishes processing.

In step S520, the operation mode setting unit 44 sets the operation mode to the elastography mode. After operation mode setting is performed by the operation mode setting unit 44, the control unit 47 proceeds to step S521. In step S521, the target region setting unit 45 performs region setting of the elastographic region-of-interest on the basis of the input position.

With the processing described above, the practitioner can simultaneously perform setting of the operation mode and setting of the examination region merely by touching (coming in contact with) a plurality of points corresponding to the region determined as an examination target on the B-mode image 100 displayed on the display unit 41. In the fifth embodiment, the mode is set to a different flow mode corresponding to the contact maintenance state, thereby enabling further specific setting of the operation mode by a single touch operation.

With the above-described fifth embodiment, the point state determination unit 43 is configured to determine the number and position of each of inputs received by the touch panel 42 capable of simultaneously receiving inputs at a plurality of positions on the B-mode image 100 displayed on the display unit 41, the operation mode setting unit 44 is configured to set the operation mode related to the B-mode image 100 on the basis of a result of the determination including the contact maintenance state, and the target region setting unit 45 is configured to set, on the basis of the position of input, a processing target region corresponding to the set operation mode. Accordingly, it is possible to simplify setting of the operation mode of the ultrasound image and input operation corresponding to the mode.

Moreover, according to the above-described fifth embodiment, the practitioner can switch operation modes merely by maintaining the contact state, making it possible to set the operation mode with minimum posture fluctuation of the practitioner caused by operation of the touch panel 42.

### (Sixth Embodiment)

Next, a sixth embodiment of the present invention will be described. The first embodiment assumes that the flow mode is set in a case where the number of detection points is three. In the sixth embodiment, however, the mode is switched to the flow mode with different color information setting on the basis of contact pressure at a detection position even when the number of detection points is three. In the sixth embodiment, the point state determination unit 43 detects the contact pressure at an input position, from a resistance value, or the like, detected by the touch panel 42. In a case where there are more than one detection point, the point state determination unit 43 outputs any of an average value, a most frequent value, a maximum value, or a minimum value, as a detected pressure.

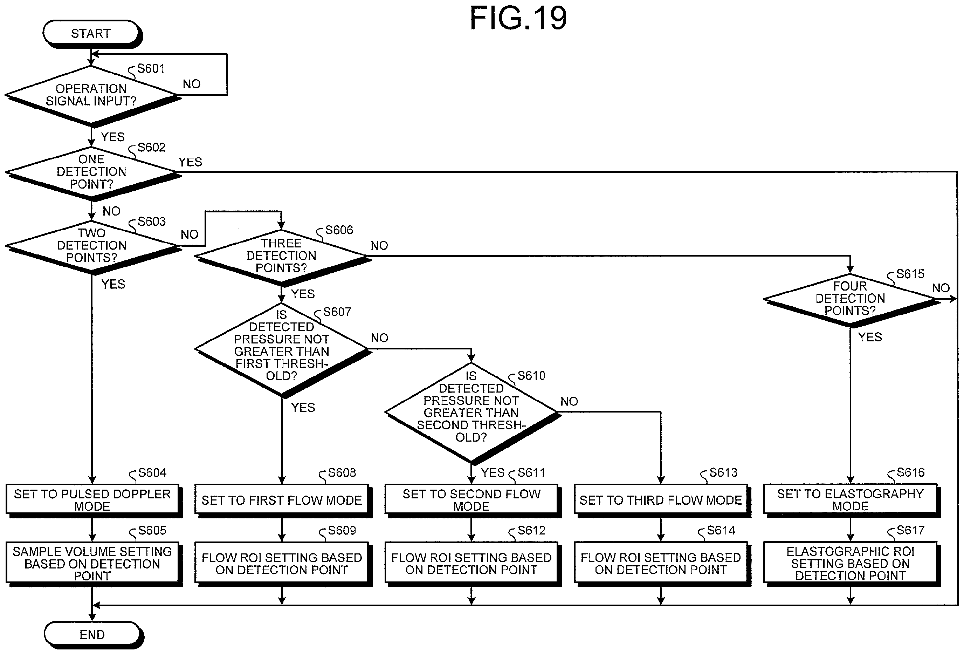

FIG. 19 is a flowchart illustrating processing performed by the operation apparatus 4 of the ultrasound observation system 1 according to the sixth embodiment. First, the control unit 47 judges whether a position signal received by the touch panel 42 is input (step S601). If the operation signal is input (step S601: Yes), the control unit 47 proceeds to step S602. In contrast, if the operation signal is not input (step S601: No), the control unit 47 returns to step S601 and repeats confirmation of input of the operation signal.

In step S602, the point state determination unit 43 initially detects the number of the inputs and a positional relationship between the input positions on the B-mode image on the basis of the position signal detected by the touch panel 42. The control unit 47 obtains the number of inputs detected by the point state determination unit 43 and judges whether the number is one, that is, whether the number of detection points is one.

When the control unit 47 judges that the number of detection points is one (step S602: Yes), the control unit 47 then judges that it is not an operation mode setting target and finishes processing. In contrast, when the control unit 47 judges that the number of detection points is not one, that is, that the number is more than one (step S602: No), the control unit 47 proceeds to step S603.

In step S603, the control unit 47 judges whether the number of detection points is two. Here, when the control unit 47 judges that the number of detection points is two (step S603: Yes), the control unit 47 proceeds to step S604.

In step S604, the operation mode setting unit 44 sets the operation mode to the pulsed Doppler mode. After operation mode setting is performed by the operation mode setting unit 44, the control unit 47 proceeds to step S605. As described above, in step S605, the target region setting unit 45 performs region setting of the sample volume on the basis of the input position.

In contrast, when the control unit 47 judges in step S603 that the number of detection points is not two (step S603: No), the control unit 47 proceeds to step S606. In step S606, the control unit 47 judges whether the number of detection points is three. Here, when the control unit 47 judges that the number of detection points is three (step S606: Yes), the control unit 47 proceeds to step S607.

In step S607, the control unit 47 judges whether the detected pressure at each of three input positions is not greater than a first threshold. Then the control unit 47 judges that the detected pressure is not greater than the first threshold (step S607: Yes), the control unit 47 proceeds to step S608 and performs operation mode setting by the operation mode setting unit 44. In step S608, the operation mode setting unit 44 sets the operation mode to the first flow mode. After operation mode setting is performed by the operation mode setting unit 44, the control unit 47 proceeds to step S609. In step S609, the target region setting unit 45 performs region setting of the flow region-of-interest on the basis of the input position.

Moreover, when the control unit 47 judges in step S607 that the detected pressure is greater than the first threshold (step S607: No), the control unit 47 proceeds to step S610. In step S610, the control unit 47 judges whether the detected pressure is not greater than a second threshold (> first threshold). Then the control unit 47 judges that the detected pressure is not greater than the second threshold (step S610: Yes), the control unit 47 proceeds to step S611 and performs operation mode setting by the operation mode setting unit 44. In step S611, the operation mode setting unit 44 sets the operation mode to the second flow mode. After operation mode setting is performed by the operation mode setting unit 44, the control unit 47 proceeds to step S612. In step S612, the target region setting unit 45 performs region setting of the flow region-of-interest on the basis of the input position.

When the control unit 47 judges in step S610 that the detected pressure is greater than the second threshold (step S610: No), the control unit 47 proceeds to step S613. In step S613, the operation mode setting unit 44 sets the operation mode to the third flow mode. After operation mode setting is performed by the operation mode setting unit 44, the control unit 47 proceeds to step S614. In step S614, the target region setting unit 45 performs region setting of the flow region-of-interest on the basis of the input position.

In contrast, when the control unit 47 judges in step S606 that the number of detection points is not three (step S606: No), the control unit 47 proceeds to step S615. In step S615, the control unit 47 judges whether the number of detection points is four. Here, when the control unit 47 judges that the number of detection points is four (step S615: Yes), the control unit 47 proceeds to step S616 and performs operation mode setting by the operation mode setting unit 44. In contrast, when the control unit 47 judges that the number of detection points is not four (step S615: No), the control unit 47 finishes processing.

In step S616, the operation mode setting unit 44 sets the operation mode to the elastography mode. After operation mode setting is performed by the operation mode setting unit 44, the control unit 47 proceeds to step S617. In step S617, the target region setting unit 45 performs region setting of the elastographic region-of-interest on the basis of the input position.

With the processing described above, the practitioner can simultaneously perform setting of the operation mode and setting of the examination region merely by touching (coming in contact with) a plurality of points corresponding to the region determined as an examination target on the B-mode image 100 displayed on the display unit 41. In the sixth embodiment, in a case where the number of detection points is three, the mode is set to a different flow mode corresponding to the detected pressure, thereby enabling further specific setting of the operation mode by a single touch operation.

With the above-described sixth embodiment, the point state determination unit 43 is configured to determine the number and position of each of inputs received by the touch panel 42 capable of simultaneously receiving inputs at a plurality of positions on the B-mode image 100 displayed on the display unit 41, the operation mode setting unit 44 is configured to set the operation mode related to the B-mode image 100 on the basis of a result of the determination including the detected pressure, and the target region setting unit 45 is configured to set, on the basis of the position of input, a processing target region corresponding to the set operation mode. Accordingly, it is possible to simplify setting of the operation mode of the ultrasound image and input operation corresponding to the mode.

In the first to sixth embodiments, the input operation is performed using the touch panel 42 on a display screen of the display unit 41. However, a touch panel may be provided on the display device 5 to perform the input operation if practitioners can operate the panel.

Moreover, while the first to sixth embodiments assume the setting of operation modes such as the pulsed Doppler mode, the flow mode, the elastography mode, the distance measurement mode, and the area measurement mode. The modes are not limited to this, however, a contrast harmonic mode or a tissue harmonic mode may be employed. Alternatively, setting of comment input node and menu display mode may be performed according to the input position.

Moreover, the first to sixth embodiments show an example in which the image processing unit 33 provided on the ultrasound observation apparatus 3 generates a display image such as a B-mode image and the calculation unit 34 calculates additional information. The configuration is not limited to this example, but may take a form in which an imaging processing unit is provided on the operation apparatus 4 and generates an image to be displayed on the display unit 41.

In the first to sixth embodiments, the ultrasound observation apparatus 3, the operation apparatus 4, and the display device 5 are provided separately. However, the ultrasound observation apparatus 3, the operation apparatus 4, and the display device 5 may be provided integrally.

Although the observation target is a living tissue in the first to sixth embodiments, an industrial endoscope for observing characteristics of material may be employed. The ultrasound observation apparatus according to the present invention is applicable both to external and internal portions of the body. The observation target may be irradiated with infrared light besides the ultrasound to transmit and receive the signal of the observation target.

In this manner, the present invention may include various forms of embodiments without deviating from the technical spirit and scope of the general inventive concept as defined in the appended claims of this invention.

### Industrial Applicability

As described above, the ultrasound observation system according to the present invention is useful in simplifying the setting of the operation mode of the ultrasound image and the input operation corresponding to the mode.

### Reference Signs List

- 1: ULTRASOUND OBSERVATION SYSTEM
- 2: ULTRASOUND ENDOSCOPE
- 3: ULTRASOUND OBSERVATION APPARATUS
- 4: OPERATION APPARATUS
- 5: DISPLAY DEVICE
- 21: ULTRASOUND TRANSDUCER
- 31: TRANSMITTING AND RECEIVING UNIT
- 32: SIGNAL PROCESSING UNIT
- 33: IMAGE PROCESSING UNIT
- 34: CALCULATION UNIT
- 35: INPUT UNIT
- 36: CONTROL UNIT
- 37: STORAGE UNIT
- 41: DISPLAY UNIT
- 42: TOUCH PANEL
- 43: POINT STATE DETERMINATION UNIT
- 44: OPERATION MODE SETTING UNIT
- 45: TARGET REGION SETTING UNIT
- 46: DISPLAY CONTROLLER

## Claims

1. An ultrasound observation system comprising:
an image processing unit configured to generate an ultrasound image based on an ultrasound signal obtained by an ultrasound endoscope configured to transmit ultrasound to a subject as an observation target and receive the ultrasound reflected from the subject;
a display unit configured to display the ultrasound image generated by the image processing unit;
a multiple input reception unit configured to simultaneously receive inputs at multiple points on the ultrasound image displayed on the display unit;
an input information determination unit configured to determine the number of the inputs and positions of the inputs, received by the multiple input reception unit;
an operation mode setting unit configured to set an operation mode related to the ultrasound image, based on a result of determination by the input information determination unit; and
a target region setting unit configured to set a target region to be processed corresponding to the operation mode set by the operation mode setting unit, based on the positions of the inputs determined by the input information determination unit.

2. The ultrasound observation system according to claim 1,
wherein the operation mode setting unit is configured to set the operation mode based on the result of determination and a display setting mode for the ultrasound image displayed on the display unit.

3. The ultrasound observation system according to claim 1 or 2,
wherein the multiple input reception unit is a touch panel provided on a display screen of the display unit.

4. The ultrasound observation system according to claim 1,
wherein the operation mode setting unit is configured to set the operation mode in accordance with the number of the inputs and a positional relationship between the inputs.

5. The ultrasound observation system according to claim 1, further comprising a control unit configured to detect at least one of the number of times for detection within a predetermined period and duration of detection at a same input position, based on the result of determination by the input information determination unit,
wherein the operation mode setting unit is configured to set the operation mode in accordance with a result of detection obtained by the control unit.

6. The ultrasound observation system according to claim 1,
wherein the input information determination unit is configured to further detect a pressure at each of the positions of the inputs, and
the operation mode setting unit is configured to set the operation mode in accordance with the number of the inputs, each of the positions of the inputs, and the pressure.
